(19) **Europäisches Patentamt / European Patent Office / Office européen des brevets**

(11) **EP 3 560 808 B1**

(12) **EUROPEAN PATENT SPECIFICATION**

(45) Date of publication and mention of the grant of the patent:
**02.08.2023 Bulletin 2023/31**

(21) Application number: **17888021.7**

(22) Date of filing: **11.12.2017**

(51) International Patent Classification (IPC):
**B62J 99/00** (2020.01)   **A61B 5/22** (2006.01)
**B62M 3/08** (2006.01)   **A61B 5/11** (2006.01)
**A61B 5/00** (2006.01)

(52) Cooperative Patent Classification (CPC):
**B62J 45/41; A61B 5/11; A61B 5/22; A61B 5/221;**
**A61B 5/4528; B62M 3/08;** A61B 5/4571;
A61B 5/4585

(86) International application number:
**PCT/JP2017/044435**

(87) International publication number:
**WO 2018/123548 (05.07.2018 Gazette 2018/27)**

(54) **JOINT-TORQUE CALCULATION DEVICE, JOINT-TORQUE CALCULATION METHOD, AND JOINT-TORQUE CALCULATION PROGRAM**

GELENKDREHMOMENTBERECHNUNGSVORRICHTUNG, GELENKDREHMOMENTBERECHNUNGSVERFAHREN UND GELENKDREHMOMENT-BERECHNUNGSPROGRAMM

DISPOSITIF DE CALCUL DE COUPLE D'ARTICULATION, PROCÉDÉ DE CALCUL DE COUPLE D'ARTICULATION, ET PROGRAMME DE CALCUL DE COUPLE D'ARTICULATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.12.2016 JP 2016251914**

(43) Date of publication of application:
**30.10.2019 Bulletin 2019/44**

(73) Proprietor: **Bridgestone Corporation**
**Tokyo 104-8340 (JP)**

(72) Inventors:
• **UCHIDA, Kazuo**
**Tokyo 104-8340 (JP)**
• **NAKANISHI, Yasuhiro**
**Ageo-shi**
**Saitama 362-0072 (JP)**

(74) Representative: **Marks & Clerk LLP**
**15 Fetter Lane**
**London EC4A 1BW (GB)**

(56) References cited:
JP-A- 2003 116 822   JP-A- 2006 110 217
JP-A- 2014 008 789   US-A1- 2007 142 177

• YUNG-SHENG LIU ET AL: "Muscles force and joints load simulation of bicycle riding using multibody models", PROCEDIA ENGINEERING, vol. 13, 16 May 2016 (2016-05-16), pages 81-87, XP028382496, ISSN: 1877-7058, DOI: 10.1016/J.PROENG.2011.05.055 [retrieved on 2011-07-26]
• YAMAZAKI HIROKI ET AL: "Joint Torque Evaluation of Lower Limbs in Bicycle Pedaling", PROCEDIA ENGINEERING, ELSEVIER, AMSTERDAM, NL, vol. 147, 9 July 2011 (2011-07-09), pages 263-268, XP029632681, ISSN: 1877-7058, DOI: 10.1016/J.PROENG.2016.06.275

**Description**

Technical Field

The present invention relates to a joint torque computation device, a joint torque computation method, and a joint torque computation program.

Background Art

Technology relating to inverse dynamic analysis is known in which the analysis is provided with information expressing a motion of a person, such as the position and speed of their skeletal structure, and then joint torques of the person are found.
For example, Patent Document 1 discloses technology to estimate joint forces and joint moments. This technology estimates joint torques and power between joints for a person. Patent Document 2 discloses technology relating to musculoskeletal modeling using finite element analysis, process integration, and design optimization. This technology performs inverse dynamic analysis on a musculoskeletal model of a person using motion capture data obtained through motion capture.
Technology is also known for analyzing the motion of a cyclist when riding a bicycle. For example, Patent Document 3 discloses technology to calculate an evaluation index of the pedaling skill on a bicycle. Patent Document 4 discloses technology for generating a real time muscle fatigue level of a cyclist, namely muscle fatigue information during pedaling a bicycle. Patent Document 5 discloses technology to analyze a movement trajectory of a cyclist's knee joints when riding a bicycle. Attention is also drawn to the disclosure of YUNG-SHENG LIU ET AL: "Muscles force and joints load simulation of bicycle riding using multibody models", PROCEDIA ENGINEERING, vol. 13 , pages 81-87, XP028382496, ISSN: 1877-7058, DOI: 10.1016/J.PROENG.2011.05.055.

Related Documents

Patent Documents

Patent Document 1: Japanese National-Phase Publication No. 2005-527004
Patent Document 2: Japanese Patent Application Laid-Open (JP-A) No. 2015-011714
Patent Document 3: JP-A No. 2014-008789
Patent Document 4: JP-A No. 2016-107093
Patent Document 5: JP-A No. 2015-091311

SUMMARY OF INVENTION

Technical Problem

The power developed by a cyclist when the cyclist is propelling a bicycle can be found by analyzing the pedaling action of the cyclist when riding the bicycle.
However, estimating a motion of a cyclist, for example the positions of joints of the cyclist, while performing a pedaling action on a bicycle, demands complex processing employing large-scale equipment such as a motion capture system Although the power developed by the cyclist may, for example, be considered dependent on the joint torque of the cyclist, the size and processing capability of bicycle mountable sensors is often limited to simple sensors, and it is difficult to find the joint torque using such simple sensors.
In consideration of the above circumstances, an object of the present invention is to estimate joint torque of the joints of a cyclist using a simple configuration.

Solution to Problem

A joint torque computation system according to the present invention is provided as claimed in claim 1.
The evaluation function may employ a function representing a strain quotient of joint power derived based on the joint torque and a joint angular velocity with respect to load applied to the pedal.
The evaluation function may be any function capable of evaluating load applied to the pedal by the cyclist. For example, using joint power as a parameter, the maximum value of the joint power, the difference between the maximum value and a minimum value thereof, and the joint power distribution may be evaluated. The joint power distribution indicates the components of the joint power waveform, and indicates, for example, the joint power distribution of one rotation of the pedal. An evaluation value to evaluate the distribution of joint power may employ a value known as a so-called root mean square (RMS) calculated using a root mean square method.

Moreover, parameters employed in an evaluation function are not limited to joint power. For example, joint torque may be employed as a parameter.

In cases in which joint torque is employed as a parameter, for example, evaluation may be performed of the joint torque maximum value, the difference between the joint torque maximum value and minimum value, and the joint torque distribution. A value calculated from the so-called RMS may, similarly to for joint power, also be employed as an evaluation value to evaluate the joint torque distribution.

The positions of the joints of the cyclist may include positions of a hip joint, a knee joint, and an ankle joint of the cyclist, and the joint torque estimation section may be configured to estimate joint torque for at least one joint out of the hip joint, the knee joint, or the ankle joint.

The joint torque computation system is configured to estimate joint torque using a cyclist model in which the cyclist in a state riding the bicycle is modeled with sites representing the hip joint, the knee joint, and the ankle joint modeled as nodes, and sites of the cyclist linking the respective nodes of the hip joint, the knee joint, and the ankle joint modeled as links.

The acquisition section may acquire the skeletal data and the structural data that has been stored in a storage section.

A joint torque computation method of the present invention is provided as claimed in claim 6.

A non-volatile storage medium storing computer program instructions according to the present invention is provided as claimed in claim 7.

Advantageous Effects

The present invention enables estimation of the joint torque of a joint of a cyclist to using a simple configuration.

BRIEF DESCRIPTION OF DRAWINGS

Fig. 1 is a schematic configuration diagram illustrating an example of a joint torque computation device according to an exemplary embodiment of the present invention.
Fig. 2 is a schematic configuration diagram illustrating a joint torque computation system according to an exemplary embodiment of the present invention and a bicycle applied with the joint torque computation system.
Fig. 3 is a schematic diagram illustrating positional relationships of joints of a lower limb of a user when the user is riding a bicycle applied with a joint torque computation system.
Fig. 4 is a schematic diagram illustrating relevant portions to express positional relationships of joints of an ankle in a pedal coordinate system.
Fig. 5 is a schematic diagram illustrating positional relationships of joints of a lower limb of a user when the user is riding a bicycle applied with a joint torque computation system.
Fig. 6 is a schematic diagram illustrating positional relationships of links of a thigh as an example of a lower limb of a user.
Figs. 7A to 7C are diagrams illustrating examples of characteristics related to a hip joint during pedaling by a user.
Fig. 8 is a schematic diagram illustrating a lower limb of a user in order to explain the principles of joint power development.
Fig. 9 is a block diagram illustrating a schematic configuration of a computer system capable of functioning as a joint torque computation system.
Fig. 10 is a flowchart illustrating an example of a flow of processing executed by a control section of a computer system according to an exemplary embodiment.

DESCRIPTION OF EMBODIMENTS

Explanation follows regarding an example of an exemplary embodiment of the present invention, with reference to the drawings. In the drawings, the arrow X, the arrow Y, and the arrow Z indicate directions corresponding to an X axis direction, a Y axis direction, and a Z axis direction in a three-dimensional coordinate system. Note that there is no limitation to the orientation applied to the present exemplary embodiment.

Power developed by a cyclist when riding a bicycle is thought to be predominantly developed by the joint torque of the joints of the cyclist. As will be described in detail later, this joint torque is derived using inverse dynamic analysis (musculoskeletal analysis) as long as there is known data regarding load applied to the pedals, data regarding the skeletal structure of the cyclist, and data regarding the structure of the bicycle. However, in cases in which a structure has been setup with the positions of some installed members changed from a bicycle structure as defined by known structural data, it is difficult to use the known load data to derive the joint torque of the joints of the cyclist. Regarding this issue, diligent research by the present inventors has led to the discovery that there is a correlation between amounts by which a variable of the bicycle structure is changed and changes in joint torque.

The present exemplary embodiment discloses a joint torque computation device that efficiently finds a change in joint torque of a joint of a cyclist when riding (when performing a pedaling action) with a bicycle structure that has been altered. Namely, in the present exemplary embodiment, the joint torque of a joint of a cyclist is efficiently found when the position of a position-adjustable member installed to a bicycle has been altered. Moreover, the present exemplary embodiment also derives a position for the member that enables the cyclist to develop maximum power.

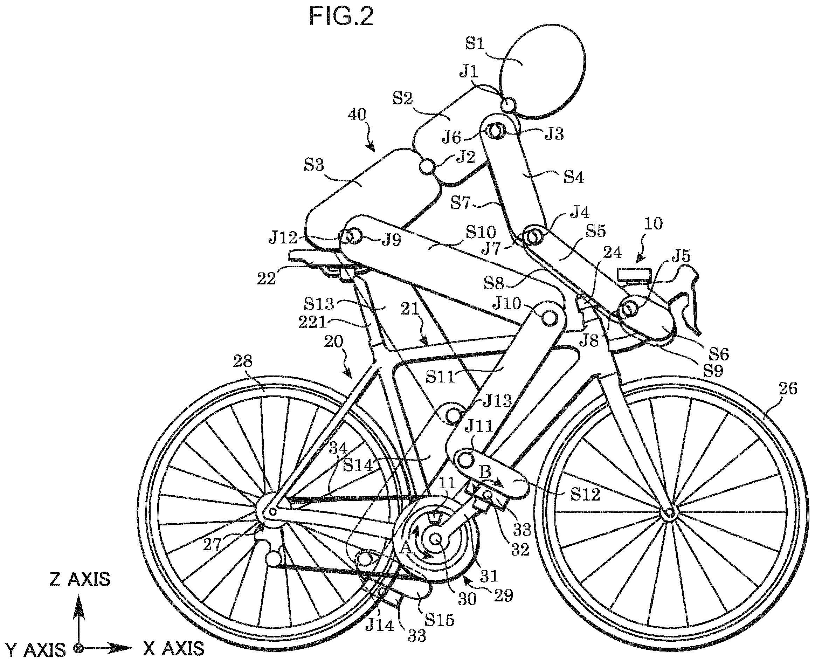

Explanation follows regarding a case in the present exemplary embodiment in which a joint torque computation system with a built in function of a joint torque computation device serves as an example of a joint torque computation device to find at least a change in joint torque. In the present exemplary embodiment, explanation follows regarding a case in which a bicycle includes a height-adjustable saddle 22 (see Fig. 2) attached to a bicycle frame specified as an example of a position-adjustable member.

Joint Torque Computation System

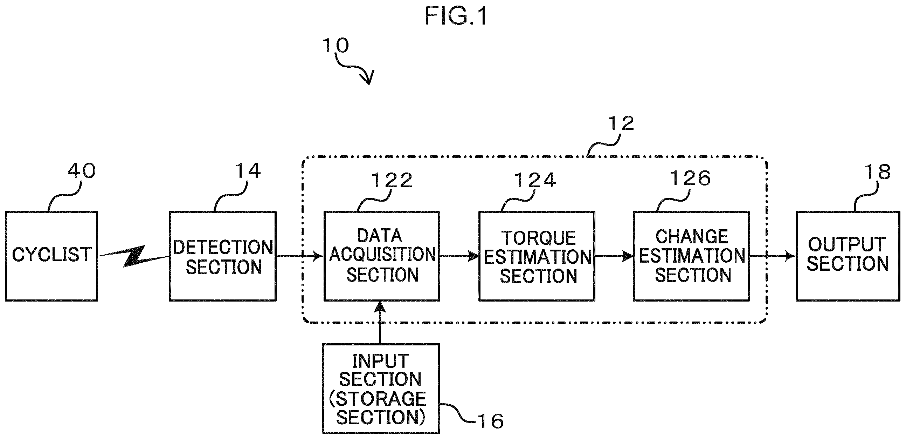

Fig. 1 illustrates an example of a schematic configuration of a joint torque computation system 10 including a joint torque computation device according to the present exemplary embodiment.

The joint torque computation system 10 according to the present exemplary embodiment includes a joint torque computation device 12, a detection section 14, an input section 16, and an output section 18. The joint torque computation device 12 also includes a data acquisition section 122, a torque estimation section 124, and a change estimation section 126.

The detection section 14 detects information relating to a bicycle when the bicycle is being ridden, and to a cyclist riding thereon. The detection section 14 of the present exemplary embodiment detects load from the cyclist acting on the pedals. For example, the detection section 14 may be configured so as to be capable of detecting at least a distribution of pedaling force during one pedal revolution as the load applied by the cyclist to a pedal attached to a crank of the bicycle (described in detail later).

The input section 16 is used to input skeletal data representing the skeletal structure of the cyclist and including the positions of joints and the inter-joint distances of the cyclist, and also structural data representing the structure of the bicycle and including an initial position of the saddle displaceably attached to the bicycle, a trajectory of a pedal rotatably attached to the bicycle, and the distance between the saddle and the pedal.

The joint torque computation device 12 estimates the joint torque of the joints of the cyclist using the load data representing the load applied to the pedal as detected by the detection section 14 and the skeletal data and structural data input via the input section 16. The joint torque computation device 12 computes the change in joint torque of the joints of the cyclist when the saddle position has been displaced. A computation result of the joint torque computation device 12 is output to the output section 18.

Specifically, the data acquisition section 122 included in the joint torque computation device 12 acquires both the load data representing the load acting on the pedals as detected by the detection section 14, and the skeletal data and structural data input via the input section 16, and outputs these data to the torque estimation section 124. The torque estimation section 124 uses the load data, skeletal data, and structural data from the data acquisition section 122 to estimate an initial joint torque of the cyclist using inverse dynamic analysis. Namely, the torque estimation section 124 uses the skeletal data, structural data, and load data for at least one pedal revolution to both estimate the motion of the cyclist, which includes the trajectory of the joints of the cyclist during a single pedal revolution with the cyclist sat on the saddle at the initial position, and to also uses inverse dynamic analysis to estimate the joint torque at each joint of the cyclist during the estimated motion of the cyclist (as described in detail later). The change estimation section 126 estimates a change in joint torque for a case in which the saddle has been displayed by a predetermined amount from the position indicated in the structural data. Namely, the change estimation section 126 uses the estimated joint torque, the load data, and the amount of displacement of the saddle away from the initial position to estimate the joint torque for a case in which the saddle has been displaced by the predetermined amount from the initial position (as described in detail later).

The output section 18 is a device such as a display device to display data representing the change in joint torque computed by the joint torque computation device 12. The output section 18 informs the cyclist of the change in joint torque.

The output section 18 is configured including a touch input-enabled liquid crystal display, and may employ a touch sensor display device capable of being used as part of the input section 16 for a cyclist 40 to input various data by touch. The output section 18 is capable of displaying information representing the joint torque, joint power, and amounts of change thereof as calculated by the joint torque computation device 12. The information display may, for example, be performed by selecting a numerical display, a symbol to indicate magnitude, a graph, or the like to be displayed.

In the change estimation section 126, the position of the saddle that would enable the cyclist to develop maximum power can be determined from the amounts of change of joint torque in plural estimations. Namely, respective changes in joint torque are estimated for cases in which the saddle has been displaced by plural different predetermined amounts from the saddle position indicated by the structural data. The saddle position enabling the cyclist to develop maximum power is determined to be a saddle position that corresponds to the amount of change at which the value of a predetermined evaluation function (described in detail later), for evaluating plural estimated amounts of change, becomes a predetermined value.

Joint Torque Change Estimation

Explanation follows regarding an estimation method for the change in joint torque when the structure of a bicycle is changed.

Note that in the present exemplary embodiment, explanation is given regarding sites relating to joints on a lower limb of the cyclist. This approach is adopted because it is thought that portions that generate the power developed by the cyclist are predominantly the lower limbs of the cyclist.

Joint Torque

First, prior to describing estimation of the amount of change in joint torque, explanation follows regarding estimation of the joint torque of the joints of the cyclist. As an example of estimation of the joint torque of the joints of the cyclist, explanation follows regarding using an inverse dynamic analysis (musculoskeletal analysis) method to derive the joint torque using the data of load applied to the pedal, the skeletal data of the cyclist including data representing a time series of motion of the skeletal structure, and the structural data for the bicycle. The joint torque of the joints of the cyclist are derived by the torque estimation section 124 illustrated in Fig. 1.

Namely, inverse dynamic analysis enables the strain on joints to be analyzed during the motion of a person. Since the motion of a cyclist is generated by rotating joints J, the strain on a joint is a torque (moment). Accordingly, joint torques (joint moments) equate to the strain on joints, and are physical quantities representing the strain on joints. Thus, in the present exemplary embodiment, a physical quantity representing the strain on a joint during the motion of a cyclist is derived by using inverse dynamic analysis.

Note that in the present exemplary embodiment, joint power is also derived for at least one joint from out of a right hip joint J9, a right knee joint J10, or a right ankle joint J1 1 on the right hand side of a lower limb. Joint power is defined as the product of the joint torque of a joint multiplied by the angular velocity of the joint (joint power = joint torque × angular velocity).

More specifically, inverse dynamic analysis can be employed to express a hip joint torque $T_{hip}$ of the right hip joint J9 by the following Equation E1. Note that a knee joint torque $T_{knee}$ of the right knee joint J10 and an ankle joint torque $T_{ankle}$ of the right ankle joint J11 can be expressed similarly, and so illustration thereof is omitted.

$$T_{hip} = \left(r_{PedalL} - r_{hip}\right) \times f_{pL}$$
$$+ \sum_{j=1,2,3} \left(I_j \dot{\omega}_j + \omega_j \times I_j \omega_j + \left(r_j - r_{hip}\right) \times m_j(\ddot{r}_i - g)\right) \quad \cdots (E1)$$

Note that Equation E1 uses the following symbols:

$T$ : torque
$f_{PL}$ : pedaling force on pedal
$I$ : inertial moment of link
$\omega$ : angular velocity of joint (rad/sec)
$\dot{\omega}$ : angular acceleration of joint (rad/sec)
$r$ : coordinate of center of mass of link
m : mass of link

$\ddot{r}$ : translational acceleration of center of mass of link

$j$ : 1 = right foot, 2 = right lower leg, 3 = right lower thigh

The left item of the first term on the right hand side of Equation E1 is an item dependent on the vector from the hip joint to the pedal, and corresponds to a vector of the moment arm from the hip joint to a pedal shaft. The right item of the first term on the right hand side is an item dependent on the pedaling force on the pedal. The second term on the right hand side is a component for moving the lower limb, and is, for example, an item dependent on the angular velocity, angular acceleration, inertial moment, and mass of each site. Namely, the second term on the right hand side represents, for example, the strain on joints to rotate a pedal 33 unloaded.

Further explanation follows regarding the method for deriving each term in Equation E1.

Bicycle and Cyclist

First, explanation follows regarding a configuration of a bicycle 20 and the cyclist 40 applicable to deriving the joint torque.

Fig. 2 is a schematic illustration of an example of a configuration of the bicycle 20 and the cyclist 40 applicable to deriving the joint torque.

Bicycle Configuration

The saddle 22 is attached via a height-adjustable seat post 221 to a frame 21 serving as a framework member configuring a bicycle frame of the bicycle 20.

A rear wheel 28 having an outer circumferential portion fitted with a tire is attached via a rear gear 27 to a rear section of the frame 21. The rear wheel 28 is configured so as to be rotatable about a Y axis, similarly to a front wheel 26.

A crank shaft 30 having a rotation axis direction running along the Y axis direction is attached to a lower section of the frame 21 so as to be coupled to a front gear 29. The crank shaft 30 is configured to rotate about the Y axis in the arrow A directions, about the Y axis. An end portion at one end of each of respective cranks (crank arms) 31 is coupled to each of the crank shafts 30. The cranks 31 are provided as a left and right pair at the two end portions of the crank shaft 30. One of the cranks 31 is attached at a position inverted by 180° about the crank shaft 30 with respect to the other of the cranks 31. A portion at the other end of each of the cranks 31 is coupled to a pedal shaft 32. Pedals 33 are attached to the pedal shafts 32 so as to be capable of rotating in the arrow B directions.

A chain 34 is entrained between the front gear 29 and the rear gear 27. The chain 34 may also be configured by a belt. When pedaling force from the cyclist 40 is imparted to the pedals 33, the pedaling force is transmitted through the pedal shafts 32 and the cranks 31 to the crank shaft 30, as a rotation force that rotates the crank shaft 30. This rotation force is transmitted to the rear wheel 28 through the front gear 29, the chain 34, and the rear gear 27, and is the driving force for propelling the bicycle 20.

Cyclist

The cyclist (referred to hereafter as the "user") gets on the bicycle 20 to propel the bicycle 20. Inverse dynamic analysis (musculoskeletal analysis) enables modeling to be performed to model an object (subject) as configured including N links (segments) S that are treated as rigid bodies, wherein N is a natural number of two or more, and including N-1 joints J, so as to enable numerical computation. In the present exemplary embodiment, the user 40 is expressed by modelling as illustrated in Fig. 2.

In the example illustrated in Fig. 2, the head of the user 40 is expressed as a link S1, and the torso and abdomen thereof are respectively expressed as links S2, S3. The right upper arm, right forearm, and right hand are respectively expressed as links S4, S5, S6, and the left upper arm, left forearm, and left hand are respectively expressed as links S7, S8, S9. The right thigh, the right lower leg, and the right foot are respectively expressed as links S10, S11, S12, and the left thigh, the left lower leg, and the left foot are respectively expressed as links S13, S14, S15.

The links are coupled to other links through joints. The link S10 corresponding to a lower limb part of the user 40 (sometimes referred to hereafter as the "right thigh S10") is coupled to the link S3 corresponding to the torso through the joint J9 (sometimes referred to hereafter as the "right hip joint J9"). The link S10 and the link S11 (sometimes referred to hereafter as the "right lower leg S11") are each coupled together by the joint J10 (sometimes referred to hereafter as the "right knee joint J10"). The link S11 and the link S12 (sometimes referred to hereafter as the "right foot S12") are each coupled together by the joint J1 1 (sometimes referred to hereafter as the "right ankle joint J1 1").

Moreover, the link S3 and the link S13 are each coupled together by the joint (left hip joint) J12, the link S13 and the link S14 are each coupled together by the joint (left knee joint) J13, and the link S14 and the link S15 are each coupled together by the joint (left ankle joint) J14.

The link S1 to the link S15 each have three positional degrees of freedom and three velocity degrees of freedom in a three-dimensional coordinate system including the X axis, the Y axis, and the Z axis. Namely, each of the link S1 to the link S15 has a total of six degrees of freedom. Similarly, the joint J1 to the joint J14 each has a total of six degrees of freedom.

In the present exemplary embodiment, joint torque is calculated for at least one joint out of the right hip joint J9, the right knee joint J10, or the right ankle joint J11 of the user 40. Note that the same method is employed for joint torque calculation on both the left and right, and so in the present exemplary embodiment explanation will be given regarding the method for joint torque calculation for the right hip joint J9, the right knee joint J10, and the right ankle joint J11 on the right side lower limb, and explanation regarding the method for joint torque calculation for the joint J12 to the joint J14 on the left side will be omitted.

Vector Derivation

Explanation first follows regarding derivation of the left part of the first term on the right hand side of Equation E1 (the vector from the hip joint to the pedal) relating to the joint torque of the user 40 when operating to propel the bicycle configured as described above.

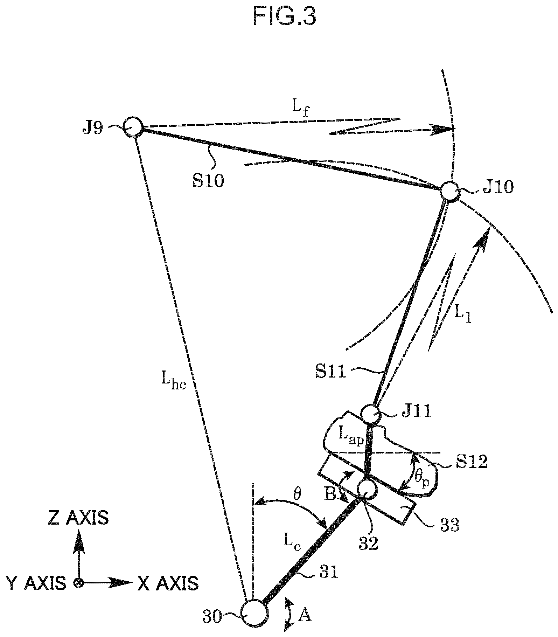

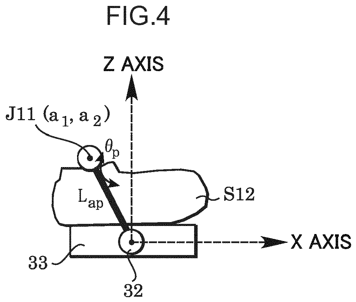

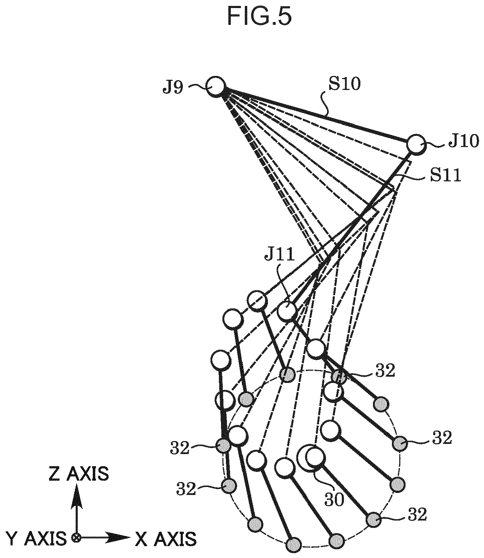

Fig. 3 schematically illustrates a positional relationship between the joints of the lower limb of the user 40. In the present exemplary embodiment, the lower limb of the user 40 is modelled in two-dimensions, as illustrated in Fig. 3. Moreover, Fig. 4 schematically illustrates the surroundings of a pedal 33 in a two-dimensional coordinate system centered on the pedal 33. Furthermore, Fig. 5 schematically illustrates a relationship between rotation of the bicycle (the cranks 31) during a pedaling action on the bicycle 20 and the positions of joints arising from movement of the lower limb of the user 40.

In the following explanation, the saddle 22 of the bicycle 20 is considered to be fixed, and a position of the adjustable saddle 22 is set as the initial value. The present exemplary embodiment employs known structural data representing the structure of respective parts of the bicycle, including an initial value for the position of the saddle 22. The foot (link S12) of the user 40 is assumed to be fixed to the pedal 33 on the bicycle 20, such that the position of the ankle (the joint position of the right ankle joint J11 in Fig. 4) does not change with respect to the pedal 33. Furthermore, the center of rotation of the crank shaft 30 is taken as the coordinate origin of the bicycle coordinate system illustrated in Fig. 3. Furthermore, although the lower limb of the user 40 is modeled in two-dimensions as illustrated in Fig. 3, when this approach is adopted, the displacement in the Y axis direction illustrated in Fig. 3 is assumed to be zero or constant for ease of explanation, and in the following explanation displacement in the Y axis direction illustrated in Fig. 3 is assumed to be zero.

Moreover, the present exemplary embodiment also employs known skeletal data regarding respective sites on the user 40. One example of the skeletal data employed includes a length Lf of the right thigh S10 from the right hip joint J9 to the right knee joint J10, a length $L_1$ of the right lower leg S11 from the right knee joint J10 to the right ankle joint J11, and a length $L_{ap}$ from the right ankle joint J11 to a central position of the pedal shaft 32. This skeletal data employs values measured in advance using a measuring instrument. Specifically, the user 40 is asked to sit on the saddle 22, and a coordinate position of the hip joint (greater trochanter) is measured. Measurement of the coordinate position may be performed using measuring instruments such as a ruler and a protractor. Alternatively, lengths and positions representing the respective sites on the skeletal structure of the user may be measured using an anthropometer or the like. Namely, the sites measured are the length Lf from the hip joint to the knee joint, the length $L_1$ from the knee joint to the ankle joint, and the length $L_{ap}$ to the position of the ankle in a pedal coordinate system.

The user 40 sits on the saddle 22 fixed at the initial value and then operates the bicycle 20. This thereby enables the joint position of the right hip joint J9 to be treated as a fixed value. Specifically, the initial position of the saddle 22 on the bicycle 20 is measured, and then, for example, at the front-rear center position and the left-right center position of the saddle 22, a position at, for example, 30 mm above the top face of the saddle 22 may be taken as the joint position of the right hip joint J9.

Moreover, although the direction (angle of orientation) of the pedal 33 might conceivably change when riding (when performing a pedaling action), in the present exemplary embodiment, the joint position of the ankle joint is calculated as a function of the crank angle based on statistical data (described in detail later). Note that a configuration may be adopted in which the angle of orientation of the pedal 33 is detected, and the joint position of the right ankle joint J11 is calculated based on angle of orientation information indicating the angle of orientation detected.

The skeletal data is input to the input section 16 as "user model information" representing the user 40 as a modeled object. The information input to the input section 16 may be stored in advance in a storage section, so as to be input from the storage section.

As illustrated in Fig. 3 and Fig. 4, since the cranks 31 are of a given length, a length $L_c$ from a center position of the crank shaft 30 to a center position of each of the pedal shafts 32 is constant. Accordingly, if a crank angle formed between the Z axis and the crank 31 is denoted θ, and the clockwise direction when viewing Fig. 3 is taken to be a positive direction, an X axis direction coordinate position $X_p$ and a Z axis direction coordinate position $Z_p$ at the rotation position of the pedal shaft 32, in a two-dimensional coordinate system with the crank shaft 30 at the origin, can be derived using the trigonometric function as expressed in Equation E2.

$$\begin{bmatrix} x_p \\ z_p \end{bmatrix} = L_c \begin{bmatrix} \sin\theta \\ \cos\theta \end{bmatrix} \quad \cdots (\text{E2})$$

The position of the right ankle joint J11 can be derived from the derived rotation position (coordinate position $X_p$, $Z_p$) of the pedal shaft 32. Namely, the length from the rotation position (coordinate position $X_p$, $Z_p$) of the pedal shaft 32 to the position of the right ankle joint J11 is constant. However, the direction (angle of orientation) of the pedal 33 might conceivably change in a regular fashion when riding (when performing a pedaling action). Accordingly, in the present exemplary embodiment a function of crank angle based on statistical data, such as that illustrated in Equation E3, is employed to derive the position (coordinate position $X_a$, $Z_a$) of the right ankle joint J11. Note that in Equation E3, sin θp and cos θp are functions of crank angle, with the clockwise direction when viewing Fig. 3 being taken to be a positive direction for angle θp.

$$\begin{bmatrix} x_a \\ z_a \end{bmatrix} = \begin{bmatrix} x_p \\ z_p \end{bmatrix} + \begin{bmatrix} \cos(\theta_p) & \sin(\theta_p) \\ -\sin(\theta_p) & \cos(\theta_p) \end{bmatrix} \begin{bmatrix} a_1 \\ a_2 \end{bmatrix}$$
$$= L_c \begin{bmatrix} \sin\theta \\ \cos\theta \end{bmatrix} + \begin{bmatrix} \cos(\theta_p) & \sin(\theta_p) \\ -\sin(\theta_p) & \cos(\theta_p) \end{bmatrix} \begin{bmatrix} a_1 \\ a_2 \end{bmatrix} \quad \cdots (\text{E3})$$

The position of the right knee joint J10 can be derived geometrically from the derived position (coordinate position $X_a$, $Z_a$) of the right ankle joint J11 and joint position of the right hip joint J9. Namely, since the joint position of the right hip joint J9 is a fixed value, for the joint position (coordinate position $X_p$, $Z_p$) of the right knee joint J10, the position can be derived for the point of intersection between a line segment of the length $L_1$ of the right lower leg S11 from the right ankle joint J11, and a line segment of the length Lf of the right thigh S10 from the right hip joint J9, and this point of intersection employed as the joint position of the right knee joint J10.

The ones of the respective joints and the center position of the pedals 33 can be derived in the manner described above.

Pedaling Force on Pedals

Next explanation follows regarding how the pedaling force on the pedals is derived, this being the right part of the first term on the right hand side of Equation E1. The pedaling force on the pedals is derived in association with the rotation positions of the pedal shafts 32. Note that the angular velocity of the crank angle θ is assumed to be constant in this case.

The detection section 14 detects the pedaling force acting on the pedal shaft 32 through the pedal 33. Namely, a pedaling force detection sensor (for example a three component force meter or a six component force meter) configuring the detection section 14 detects the magnitude and direction of pedaling force in a two-dimensional coordinate system. The magnitude and direction of the pedaling force are detected as a distribution over a cycle corresponding to the cycle of one revolution of the pedal 33.

Accordingly, a distribution of pedaling force information for a single revolution of the crank 31 (the magnitude and direction of the pedaling force) is measured and divided into a predetermined number of divisions, for example 100 divisions. This enables force acting in the X axis direction and force acting in the Z axis direction to be derived for every angle of one hundredth of a rotation (360/100). The pedaling force information may also be expressed as a function of crank angle θ. Moreover, for example, measuring the time taken for a single cycle enables the pedal revolutions per minute to be calculated, thereby enabling the angular velocity to also be derived.

In order to detect information relating to the pedaling force of the cyclist more accurately, plural sensors may be provided to function as the detection section 14. For example, the detection section 14 may include a first detector to detect the rotation position of the crank 31, a second detector to detect the magnitude or the magnitude and direction of the pedaling force acting on the pedal shaft 32, and a third detector to detect the angle of orientation (tilt angle) of the pedal 33 with respect to the pedal shaft 32.

A magnetic or optical rotation (revolution speed) detection sensor may be employed as an example of the first detector. The first detector is mounted to the front gear 29, and detects a rotation position of the crank 31 as it rotates about the crank shaft 30. Detecting the rotation position of the crank 31 also enables the rotation position of the pedal shaft 32 to be detected by detecting the rotation position of the crank 31, since the length of the crank 31 (more precisely, the dimension from the center of the crank shaft 30 to the center of the pedal shaft 32) is known in the length direction of the crank 31.

A pedaling force detection sensor (pedaling-force meter) may be employed as an example of the second detector. The second detector is mounted to the pedal shaft 32, and detects the magnitude or the magnitude and direction of the pedaling force from the lower limb of the cyclist (user) 40 acting on the pedal shaft 32 through the pedal 33 in the two-dimensional coordinate system including the X axis and the Z axis. A pressure sensor mounted to the pedal 33 may also be employed as the second detector.

An inertia sensor may be employed as an example of the third detector. The third detector is mounted to either the pedal 33 or the pedal shaft 32 and detects the angle of orientation (tilt angle) of the pedal 33 with respect to the pedal shaft 32.

Lower Limb Component

Explanation next follows regarding how to derive components (for example angular velocity, angular acceleration, inertial moment, and mass at each site) for moving the lower limb, i.e. the second term on the right hand side of Equation E1. As an example, explanation follows regarding a case in which kinematic quantities are derived for each link.

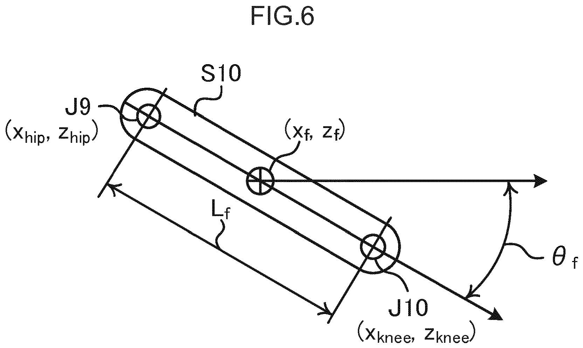

Fig. 6 schematically illustrates positional relationships of a link of the thigh (right thigh S10) serving as an example of a lower limb of the user 40.

As illustrated in Fig. 6, the angle ($\theta_f$) formed between the right thigh S10 and the X axis can be derived using Equation E4 below by employing the joint position ($x_{hip}$, $z_{hip}$) of the right hip joint J9 and the joint position ($x_{knee}$, $z_{knee}$) of the right knee joint J10 therein. The position of the center of mass of the right thigh S10 can also be derived using Equation E5 below.

$$\theta_f = \alpha_f tan2(x_{knee} - x_{hip}, \; z_{knee} - z_{hip}) \quad \cdots (E4)$$

$$\begin{bmatrix} x_f \\ y_f \end{bmatrix} = \begin{bmatrix} x_{hip} \\ y_{hip} \end{bmatrix} + \alpha_f \left( \begin{bmatrix} x_{knee} \\ y_{knee} \end{bmatrix} - \begin{bmatrix} x_{hip} \\ y_{hip} \end{bmatrix} \right) \quad \cdots (E5)$$

Wherein: $\alpha_f$ is a proportion to express the location of the position of the center of mass, and an existing database may be employed therefor. An example of such an existing database is given in the document "Estimation of Inertia Properties of Body Segments in Japanese Athletes (forms and kinematic measurement)" by Michiyoshi AE, Haipeng TANG, Takashi YOKOI, Biomechanisms Vol. 11 (1992), pp 23 to 33.

For example, the position of the center of mass of the right thigh S10 changes continuously when riding. The geometric calculations of structure as described above may thus be employed to derive the position of the center of mass that is changing continuously with time, and the angular velocity, angular acceleration, and acceleration then derived by differentiating with respect to time as expressed in Equations E6.

$$\alpha_f x_f := (x_f(n+1) - 2\,x_f(n) + x_f(n-1))/dt^2$$

$$\alpha_f z_f := (z_f(n+1) - 2\,z_f(n) + z_f(n-1))/dt^2$$

$$\dot{\omega}_f := (\theta_f(n+1) - \theta_f(n) + \theta_f(n-1))/dt^2$$

$$\omega_f := (\theta_f(n) - \theta_f(n-1))/dt \quad \cdots (E6)$$

where,

$$\alpha_f x_f \; \rightarrow \; \alpha_{xf} \; , \alpha_f z_f \; \rightarrow \; \alpha_{zf}$$

Note that in cases in which there is a variation (fluctuation) present in the data values derived for any of the angular velocity, angular acceleration, or acceleration, processing is preferably performed using an appropriate filter (for example, a 10 Hz fourth-order Butterworth filter).

The mass and inertial moment of each site are derived using known methods while employing the body weight and the lengths between each joint of the user 40. The calculation formulae in the above document may be employed as an example of a known method.

Change in Joint Torque Arising from Saddle Position Changes

The joint torque of the joints of the user 40 can be estimated, namely, the joint torque of each joint can be derived, as described above.

It is known from experience that in cases in which, due to the idiosyncrasies of the user 40 or the like, differences arise in a pattern of the action adopted to develop power, changing part of the structure of the bicycle 20, for example adjusting the position of the saddle 22, enables the maximum development of power by the user. However, estimating joint torque for every change to part of the structure of the bicycle 20 using inverse dynamic analysis is not realistic due to the enormous computational load that this would incur. Namely, although the joint torque can be derived for each joint of the user 40 at the initial position of the saddle 22, it is difficult to derive the changes in the joint torques arising when the saddle 22 has been displaced from the initial position.

Diligent research by the inventors has focused on the fact that the first term on the right hand side of Equation E1 is the component of power output, and the second term on the right hand side of Equation E1 is the component employed to move the lower limb. As the result the inventors have discovered that an interdependent relationship exists between the amount of change when part of the bicycle structure is changed and the amounts of change in joint torque. Namely, the present exemplary embodiment enables the change in joint torque to be derived simply. This enables derivation of the structure of the bicycle 20 that enables the maximum development of power by the user, namely derivation of the optimum position for the saddle 22. The change in joint torque of the user 40 is derived by the change estimation section 126 illustrated in Fig. 1.

Detailed explanation follows regarding how the change in joint torque is derived. Equation E1 can be expressed using Equation E7 below.

$$T = J^T f + K \quad \cdots \text{(E7)}$$

Specifically, the first term on the right hand side of Equation E1, which is the component of power output, is a component that is the product of component $J^T$ (position) predominated by the geometric structure, multiplied by component f predominated by the pedaling force on pedal. The second term on the right hand side of Equation E1, which is the component employed to move the lower limb, is a component K representing the strain on joints when rotating the pedal 33 in an unloaded state, and is thought to make little contribution to the joint torque. Note that J is a Jacobian (Jacobian matrix).

Taking Equation E7, then a case in which the structure of the bicycle 20) has been changed, namely the position of the saddle 22 has been changed, can be expressed by the following polynomial Equation E8.

$$T + \Delta T = (J^t + \Delta J^t)(f + \Delta f) + (K + \Delta K) \quad \cdots \text{(E8)}$$

The symbols in Equation E8 are expressed by the following Equation E9, wherein $h_0$ is the initial position of the saddle 22 and $\Delta h$ is the change from the initial position.

$$\begin{aligned}
\Delta T &:= T(h_0 + \Delta h)\text{-}T(h_0) \\
\Delta J &:= J(h_0 + \Delta h)\text{-}J(h_0) \\
\Delta f &:= f(h_0 + \Delta h)\text{-}f(h_0) \\
\Delta K &:= K(h_0 + \Delta h)\text{-}K(h_0)
\end{aligned} \quad \cdots \text{(E9)}$$

Equation E7 and Equation E8 can be used to give an approximation for the change in joint torque $\Delta T$ as expressed by the polynomial Equation E10 below.

$$\Delta T \approx \Delta J^t f + J^t \Delta f + \Delta K \quad \cdots \text{(E10)}$$

As the result of diligent research, the inventors have observed that there is a tendency for the second term and the third term on the right hand side of Equation E10 to cancel each other out. This has led to the conclusion that Equation E9 can be expressed as the following Equation E11, and the change in joint torque ($\Delta T$) can be approximated to a value obtained by multiplying the change in geometric structure ($\Delta J^T$) by the pedaling force on pedal (f).

$$\Delta T \approx \Delta J^t f \quad \cdots (E11)$$

Accordingly, the joint torques when the height of the saddle 22 has been changed by a change $\Delta h$ from the initial position $h_0$ can be expressed as a function of the height of the saddle 22 as expressed by Equation E12 below.

$$T(h) \approx T_0 + \Delta J^t f \quad \cdots (E12)$$

In Equation E12, the initial value $T_0$ of the joint torque is expressed as Equation E13 below.

$$T_0 = J^t f + K \quad \cdots (E13)$$

Note that in the present exemplary embodiment, the position of a member (the height of the saddle 22) to be derived that enables maximum power development by the user 40 from the joint torques and the changes in joint torque derived for a case in which the height of the saddle 22 has been changed.

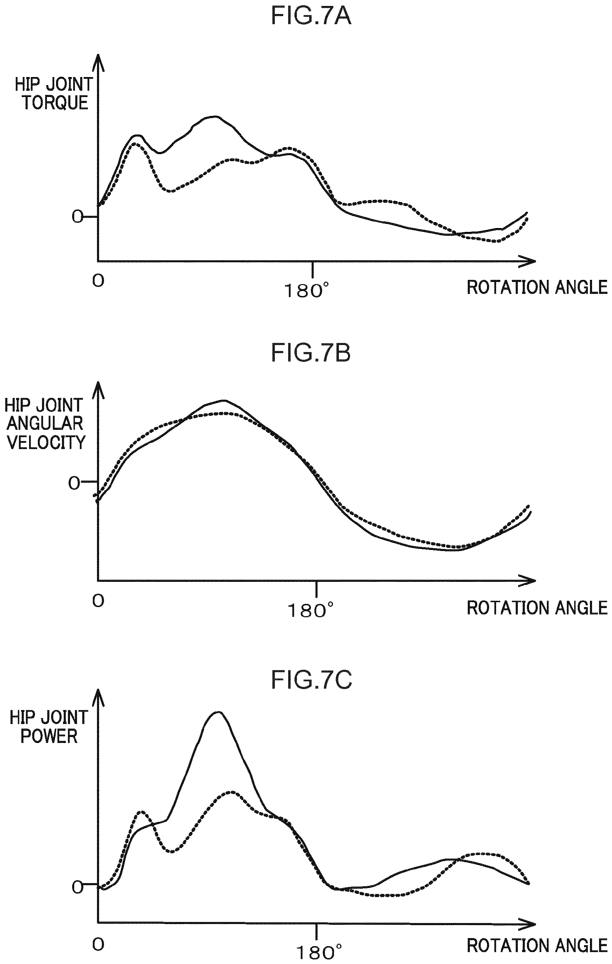

Figs. 7A to 7C illustrate examples of characteristics related to the hip joint when two users 40 each performed a pedaling action on the bicycle 20. Fig. 7A illustrates hip joint torque characteristics, Fig. 7B illustrates hip joint angular velocity characteristics, and Fig. 7B illustrates hip joint power characteristics. In Figs. 7A to 7C, the solid lines represent a so-called advanced user (referred to hereafter as the user 40pro), and the dotted lines represent a so-called beginner user (referred to hereafter as the user 40ama).

As illustrated in Fig. 7B, the hip joint angular velocity does not differ greatly between the user 40pro and the user 40ama. Namely, on initial appearances, the user 40pro and the user 40ama may be estimated to be pedaling with a similar action to each other. However, as illustrated in Fig. 7A, for the hip joint torque, the user 40pro generates hip joint torque that is a substantially average amount on the down-stroke of pedaling, whereas there are large fluctuations thereat to the hip joint torque of the user 40ama. As illustrated in Fig. 7C, for hip joint power, the user 40pro generates a large hip joint power on the down-stroke of pedaling, whereas there are large fluctuations thereat for the user 40ama. Namely, although the user 40pro is presumed to be developing their maximum power, the user 40ama is presumed to be unable to develop their maximum power, and to have room for improvement.

In order to address this, the present exemplary embodiment evaluates the pedaling performance of each of the users 40 for changes to the position of a member (the height of the saddle 22) in order to derive the position of a member (the height of the saddle 22) that enables the user 40 to develop their maximum power. Specifically, a physical quantity significant to the user 40 in relation to joint torque is identified, and pedaling performance is evaluated by determining the magnitude of the identified physical quantity. An example of pedaling performance evaluation in the present exemplary embodiment is performed by taking a joint power contribution quotient with respect to power transmitted to the pedal 33, namely pedaling power (propulsion power), as a pedaling performance index (performance measure).

Note that although explanation is given regarding an example of the present exemplary embodiment in which pedaling performance is evaluated using a pedaling performance index (performance measure), there is no limitation employing a pedaling performance index (performance measure). Namely, although explanation is given regarding a case in the present exemplary embodiment in which a function representing a strain quotient (contribution quotient) of joint power, derived based on the joint torque and the joint angular velocity against the load on the pedal, is employed as an evaluation function, the evaluation function is not limited thereto.

Namely, explanation is given regarding an example of a case in which load applied to the pedal by the cyclist is evaluated. However, as another example the joint power may be employed as a parameter, and evaluation performed of the maximum value of the joint power, the difference between the maximum value and a minimum value thereof, and the joint power distribution. The joint power distribution indicates the components of the joint power waveform, and indicates, for example, the joint power distribution of one rotation of the pedal. An evaluation value to evaluate the distribution of joint power may employ a value known as a so-called root mean square (RMS) calculated using a root mean square method. Moreover, parameters employed in an evaluation function are not limited to joint power. For example, joint torque may be employed as a parameter. In cases in which joint torque is employed as a parameter, for example, evaluation may be performed of the joint torque maximum value, the difference between the joint torque maximum value and minimum value, and the joint torque distribution. A value calculated from a RMS may, similarly to for joint power, also be employed as an evaluation value to evaluate the joint torque distribution.

Pedaling performance evaluation corresponds to finding the optimum value for an object function obj. In the present exemplary embodiment, an evaluation function represented by Equation E14 below is employed as the object function obj. Note that the evaluation function represented by Equation E14 expresses a strain quotient (contribution quotient) of hip joint power with respect to pedal power. A contribution quotient of knee joint power and a contribution quotient of ankle joint power can be derived similarly to the evaluation function represented by Equation E14. These performance measures are derived by the change estimation section 126 illustrated in Fig. 1.

$$R_{hip} := \frac{(1/t_{cycle}) \int_0^{t_{cycle}} T_{hip}\,\omega_{hip}\,dt}{(1/t_{cycle}) \int_0^{t_{cycle}} v_{pedal}^T f_{pedal}\,dt} \qquad \cdots (E14)$$

Wherein in Equation E14, $t_{cycle}$ represents the time required for a single revolution of the pedal 33. Accordingly, Equation E14 represents a ratio of the average values of the components for a joint with respect to the average value of pedal power for one of the pedals 33.

Joint Power

Explanation follows regarding the derivation of joint power in pedaling performance evaluation.
In the present exemplary embodiment, the joint torque is derived and then the joint power is calculated. The joint torque is calculated for the lower limb system of the user 40 when performing a pedaling action on the bicycle 20.

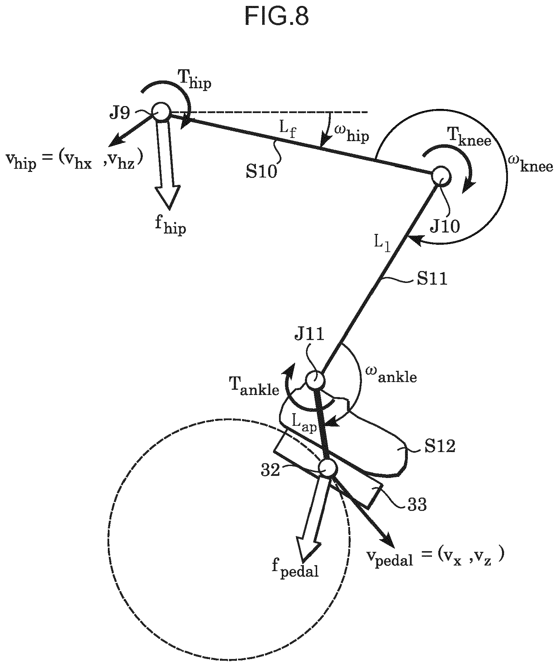

Fig. 8 schematically illustrates the lower limb of the user 40 in order to explain the origin of joint power developed by the user 40.
The user 40 transmits power to the pedal 33 of the bicycle 20. The power transmitted to the pedal 33 is transmitted to the chain 34 in sequence through the pedal shaft 32, the crank 31, the crank shaft 30, and the front gear 29. This power is then transmitted onward to the rear wheel 28 through the rear gear 27, resulting in the driving force of the bicycle 20. The origin of the power transmitted to the pedal 33, namely the origin of the pedal power (propulsion power) development, can be derived from the polynomial equation expressed by Equation E15 in which terms for each joint have been separated out from an equation of motion for the user 40.

$$v_{pedal}^T f_{pedal} = v_{hip}^T f_{hip} + T_{hip}\,\omega_{hip} + T_{knee}\,\omega_{knee} + T_{ankle}\,\omega_{ankle}$$

$$\cdots (E15)$$

Wherein the meaning of each of the symbols employed in the Equation is as follows:

$v_{pedal}$ : translational velocity of pedal (rad/sec)

$f_{hip}$ : reaction force on the user from the saddle (N)

$T_{hip}$ : joint torque of hip joint (Nm)

$T_{knee}$ : joint torque of knee joint (Nm)

$T_{ankle}$ : joint torque of ankle joint (Nm)

$\omega_{hip}$ : angular velocity of hip joint (rad/sec)

$\omega_{knee}$ : angular velocity of knee joint (rad/sec)

$\omega_{ankle}$ : angular velocity of ankle joint (rad/sec)

$v_{hip} = (v_{hx}, v_{hz})$:     translational velocity of hip joint (rad/sec)

The first term on the right hand side of Equation E15 is power from translational motion of the hip joint, in this case the right hip joint J9. The second term on the right hand side is the hip joint power of the right hip joint J9. The third term on the right hand side is the knee joint power of the right knee joint J10. The fourth term on the right hand side is the ankle joint power of the right ankle joint J11. Namely, each joint power is expressed by the product of joint torque and angular velocity.

The reaction force on the user 40 from the saddle 22 due to the upper body of the user 40, and is a force acting through the hip joint on the right lower limb. More precisely, the power due to translational motion of the hip joint in the first term on the right hand side includes a component arising from the user 40 using their upper body weight to press their foot downward through their hip joint, a component to move the right foot through the movement of the left foot, a component of power transmitted to the lower limb as a reaction to pushing or pulling strongly on the handlebars, and the like. Namely, this power is not power developed by the hip joint, but is a component of power from sites other than the lower limb that affects the pedal 33 through the hip joint.

The hip joint power contribution quotient with respect to the pedal power is expressed by Equation E16 below.

$$v_{hip} f_{hip} \diagup v_{pedal} f_{pedal} \quad \cdots (E16)$$

The contribution quotient of power due to translational motion of the hip joint has been found by experimentation to not be significant in pedal power.

Pedal power is the amount of energy converted per unit time, and is therefore a physical quantity that continuously changes with time. Accordingly, the contribution quotient of the respective components of the first term on the right hand side to the fourth term on the right hand side of Equation E15 with respect to the pedal power therefor also change continuously with time.

Thus using Equation E14 to calculate a ratio of the average values of each component with respect to the average value of pedal power for a predetermined rotation angle of the pedal 33 (per single cycle) enables a performance measure to be derived that quantifies the joint power. The ratio in Equation E14 of the average value of hip joint power with respect to the average value of the pedal power is calculated to quantify a performance measure related to hip joint power. The power of other joints can also be quantified using a similar technique. Moreover, the predetermined rotation angle might be an angle within a single revolution of the crank 31, such as, for example, an angle of 180° from the top dead center to the bottom dead center of the crank 31, might be one revolution of the crank 31, or might be plural revolutions such as two or more revolutions.

The position of the saddle 22 where the performance measure is greatest is accordingly derived as the position of a member (the height of the saddle 22) that would enable the user 40 to develop their maximum power. For example, in cases in which the right hip joint power is evaluated, the position of the saddle 22 where the performance measure related to the hip joint as expressed by Equation E14 is greatest can be presented to the user 40 as the position enabling a large hip joint power to be developed by the right hip joint J9.

Computer System

The joint torque computation system 10 illustrated as an example in Fig. 1 may be implemented by a computer system including a control section configured by a generic computer.

Computer System Configuration

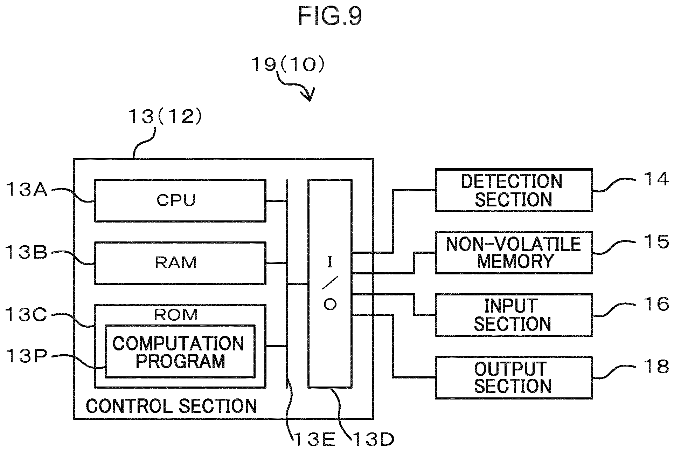

Fig. 9 schematically illustrates a computer system 19 that can be made to function as the joint torque computation system 10. Note that the computer system 19 may be applied to a cycle computer mounted to the bicycle 20.

The computer system 19 includes a control section 13 that functions as the joint torque computation device 12. The control section 13 is configured by a computer including a CPU 13A, RAM 13B, ROM 13C, and an I/O 13D. The CPU 13A, the RAM 13B, the ROM 13C, and the I/O 13D are connected to a bus 13E so as to be capable of exchanging data and commands with each other. A computation program 13P is stored in the ROM 13C. The computation program 13P includes processes to cause the control section 13 to function as the data acquisition section 122, the torque estimation section 124, and the change estimation section 126 of the joint torque computation device 12.

The detection section 14, the input section 16, and the output section 18 are connected to the I/O 13D. In the example in Fig. 9, non-volatile memory 15 serving as a storage section is connected to the I/O 13D and pre-stored with information including both the user model information including the skeletal data input via the input section 16, and also the structural data for the bicycle 20.

In the control section 13, the CPU 13A reads the computation program 13P stored in the ROM 13C and expands the computation program 13P in the RAM 13B. The control section 13 then operates as the joint torque computation device 12 by executing the expanded computation program 13P.

Computer System Operation

Explanation next follows regarding specific processing performed by the control section 13 of the computer system 19 according to the present exemplary embodiment.

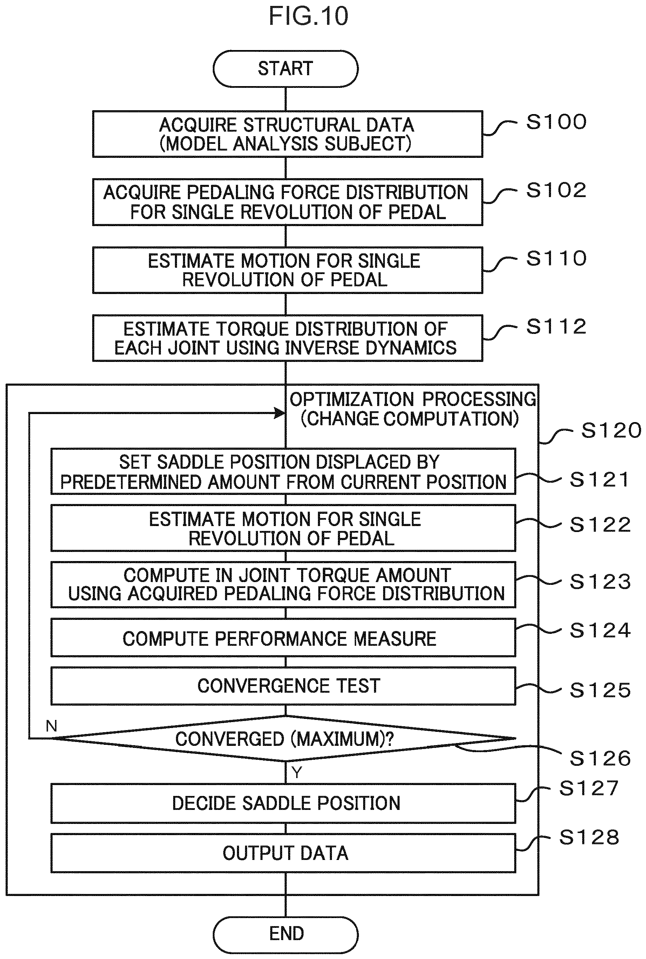

Fig. 10 is a flowchart illustrating an example of a flow of processing executed by the control section 13 of the computer system 19 according to the present exemplary embodiment. Note that the processing of Fig. 10 starts when a non-illustrated power switch of the computer system 19 has been switched ON. Alternatively, the processing of Fig. 10 may start when a command instructed by the user 40 has been input via the input section 16.

At step S100, the CPU 13A acquires the skeletal data of the user 40 and the structural data of the bicycle 20 from the non-volatile memory 15. Moreover, at step S100, the skeletal data and the structural data are employed to model the analysis subject of the user 40 and the bicycle 20. At the next step S102, data is acquired for the pedaling force for a single revolution of the pedal, as detected by the detection section 14. The data related to the pedaling force for the one revolution of the pedal acquired at step S102 corresponds to the component f predominated by the pedaling force in the first term on the right hand side of Equation E7. The processing of step S100 and step S102 corresponds to the function of the data acquisition section 122 of the joint torque computation device 12 illustrated in Fig. 1.

At the next step S110, an action of a single revolution of the pedal 33 by the user 40 is estimated by geometric calculation. At the next step S112, the distribution of the joint torque of each joint during the single revolution of the pedal 33 is derived by computation processing using inverse dynamic analysis (see also Equation E1). Note that the motion of the single revolution of the pedal 33 by the user 40 estimated at step S110 corresponds to the component $J^T$ predominated by the geometric structure in the component of power output of the first term on the right hand side of Equation E7, and to the component K for moving the lower limb of the second term on the right hand side of Equation E7. The processing of step S110 and step S112 corresponds to the function of the torque estimation section 124 of the joint torque computation device 12 illustrated in Fig. 1.

Next, at step S120, processing to optimize joint torque is executed. The processing of step S120 corresponds to the function of the change estimation section 126 of the joint torque computation device 12 illustrated in Fig. 1. At step S120, the change in joint torque is derived for a case in which at least the position of the saddle 22 has been changed. The optimum position for the saddle 22 may also be derived at step S120. More precisely, at step S121, the position of the saddle 22 is set for when displaced by a predetermined amount from the current position. In initial processing, the position of the saddle 22 is set at a predetermined displacement (change $\Delta h$ from the initial position) from the initial value (initial position $h_0$ of the saddle 22) in the structural data of the bicycle 20 acquired at step S100. At the next step S122, the motion of the user 40 for a single revolution of the pedal 33 with the position of the saddle 22 displaced by the predetermined amount is estimated similarly to at step S110. The motion of the user 40 estimated at step S122 corresponds to the change in geometric structure ($\Delta J^T$) in Equation E11. At the next step S123, the data (pedaling force distribution) for the pedaling force during a single revolution of the pedal acquired at step S102 is employed to compute changes in joint torque. Namely, at step S123, Equation E11 is employed to derive, as the change in joint torque ($\Delta T$), a value obtained by multiplying the structural change ($\Delta J^T$) by the pedaling force (f) on the pedal.

At the next step S124, the performance measure to evaluate pedaling performance is computed. Namely, the strain quotient of hip joint power with respect to the pedal power, for example, is computed using the evaluation function of Equation E14 to derive the performance measure. At the next step S125, a convergence test is executed on the performance measure derived at step S123. This convergence test is processing to determine whether or not the performance measure derived at step S123 is the maximum value out of performance measures derived thus far. At the next step S126, affirmative determination is made in cases in which the test result has converged (is at a maximum) at step S125, and processing transitions to step S127. In cases in which determination is negative at step S126, processing returns to step S121, and the above processing is executed with the position of the saddle 22 displaced by a predetermined amount from its current position.

The processing to determine the maximum value of the performance measure may be performed by selecting from out of plural performance measures that have been derived over a predetermined adjustment range of the saddle 22. Alternatively, this processing may be performed by monitoring the magnitude of the slope of change from the previously derived performance measure, finding an inflection point in the performance measurement characteristics, and taking the value corresponding to the inflection point as the performance measure.

Next, at step S127, the position of the saddle 22 corresponding to the joint torque change giving the maximum value of the performance measure is decided as the height of the saddle 22 enabling the user 40 to develop their maximum power, and information representing the decided height of the saddle 22 is output to the output section 18 at the next step S128. In this manner, the height of the saddle 22 enabling the user 40 to develop their maximum power can be presented to the user 40 via the output section 18.

Presentation to the user 40 via the output section 18 may be configured by the change in joint torque alone. In such cases, the processing of step S124 and step S 125 may be skipped in a configuration in which determination processing is performed to determine whether or not the determination processing of step S 126 has been executed a predetermined number of times.

Test Examples

Table 1 below illustrates the results of performing processing to optimize the position of saddle 22 for different users 40 using the joint torque computation system 10 according to the present exemplary embodiment.

Table 1

| Saddle Height Change (mm) from Reference Height ($h_0$) | Object Function (Hip Joint Strain Quotient) (%) | |
|---|---|---|
| | User 1 | User 2 |
| -16 | 38 | 20.7 |
| -14 | 39.5 | 24 |
| -12 | 40.6 | 26.6 |
| -10 | 41.4 | 29 |
| -8 | 41.8 | 31.1 |
| -6 | 42 | 32.7 |
| -4 | 41.9 | 33.9 |
| -2 | 41.4 | 34.8 |
| 0 | 40.6 | 35.1 |
| 2 | 39.5 | 35 |
| 4 | 38 | 34.7 |
| 6 | 36.3 | 34 |
| 8 | 34.3 | 32.8 |

Table 1 lists the test results for two users 40, namely a user 1 with a height of 171 cm and a body weight of 62 kg, and a user 2 with a height of 165 cm and a body weight of 55 kg.

The pedaling actions thereof were measured for one minute in a steady state at 90 revolutions per minute at 240 W on a static bike.

Measurements are taken using motion capture with markers affixed to the hip joint, knee joint, ankle joint, and pedal. A three component force meter was disposed on the pedal so as to acquire a time series of data therefrom. This positional and force data was averaged across approximately 90 revolutions to give data for a single revolution. The results of subjecting this data to the optimization processing described above (torque prediction and object function calculation) enabled optimal saddle heights to be derived that were different for each of the user 1 and the user 2.

As is apparent from the test results illustrated in Table 1, the optimization of saddle position using the joint torque computation system 10 has ample practical utility.

Other Exemplary Embodiments

For example, although in the above exemplary embodiment the present invention is applied to measurement (calculation) of joint torque and joint power measurement (calculation) of a lower limb of a user riding a bicycle, examples not falling under the scope of the claims may be applied to measurement of joint torque and joint power of lower limbs and upper limbs of a user rowing a race boat.

Although the user is a human in the above exemplary embodiment, the present invention may, for example, be applied to a humanoid robot having link and joints equivalent to those of a human, or may be applied to a robot having link and joints corresponding to those of a lower limb. The present invention may of course also be applied to measurement of joint torque and joint power of an animal.

Moreover, although explanation has been given regarding an example in the above exemplary embodiment in which a display device is applied as the output section, the output section may be configured by an audio output device (an example not falling under the scope of the claims), or by a combination of a display device with an audio output device. Specifically, an audio output device may be configured to use audio to inform the user operating the bicycle of joint torque and joint power.

**Claims**

1. A joint torque computation system (10) comprising:

   a detection section (14) configured to detect load and pedaling force applied to a pedal by a cyclist;
   an acquisition section (122) configured to acquire

   skeletal data representing a skeletal structure of a cyclist including a position of joints of the cyclist and an inter-joint distance,
   structural data representing a structure of a bicycle and including an initial position of a saddle displaceably attached to a bicycle frame, a trajectory of a pedal rotatably attached to the bicycle frame, and a distance between the saddle and the pedal, and
   load data representing the load detected by the detection section (14), the load data including pedaling force data detected by the detection section (14)

   a joint torque estimation section (124) configured to employ the skeletal data, the structural data, and data in the load data corresponding to at least one revolution of the pedal to estimate a trajectory of a joint of the cyclist for the one revolution of the pedal when the cyclist is seated on the saddle in an initial position, and to use inverse dynamic analysis to estimate joint torque for the respective joints of the cyclist based on an estimated motion of the cyclist;
   a change estimation section (126) configured to

   set the position of the saddle for when displaced by a predetermined amount from the current position;
   estimate the motion of the cyclist for a single revolution of the pedal with the position of the saddle displaced by the predetermined amount;
   employ the data acquired for the pedaling force during a single revolution of the pedal to compute changes in joint torque,
   compute a predetermined performance measure to evaluate pedaling performance,
   execute a convergence test on the performance measure to determine whether or not the performance measure is the maximum value out of performance measures derived thus far, and, when affirmative determination is made that the test result is at a maximum, transition to the following step, but when determination is negative, return to the previous step of setting the position of the saddle, in which case the processing is executed with the position of the saddle displaced by a predetermined amount from its current position, and
   decide the position of the saddle corresponding to the joint torque change giving the maximum value of the performance measure as the height of the saddle enabling the cyclist to develop their maximum power; and

   an output section (18) configured to display the decided height of the saddle enabling the cyclist to develop their maximum power derived by the change estimation section (126).

2. The joint torque computation system (10) of claim 1, wherein the performance measure is a function representing a strain quotient of joint power derived based on the joint torque and a joint angular velocity with respect to load applied to the pedal.

3. The joint torque computation system (10) of claim 1 or claim 2, wherein:

   positions of the joints of the cyclist include positions of a hip joint, a knee joint, and an ankle joint of the cyclist; and
   the joint torque estimation section (124) is configured to estimate joint torque for at least one joint out of the hip joint, the knee joint, or the ankle joint.

**4.** The joint torque computation system (10) of claim 3, wherein joint torque is estimated using a cyclist model in which the cyclist in a state riding the bicycle is modeled with sites representing the hip joint, the knee joint, and the ankle joint modeled as nodes, and sites of the cyclist linking the respective nodes of the hip joint, the knee joint, and the ankle joint modeled as links.

**5.** The joint torque computation system (10) of any one of claim 1 to claim 4, wherein the acquisition section (122) is configured to acquire the skeletal data and the structural data that has been stored in a storage section.

**6.** A joint torque computation method comprising:

> detecting in a detection section (14) load and pedaling force applied to a pedal by a cyclist;
> acquiring (S100, S102) in an acquisition section (122) skeletal data representing a skeletal structure of a cyclist including a position of joints of the cyclist and an inter-joint distance, structural data representing a structure of a bicycle and including an initial position of a saddle displaceably attached to a bicycle frame, a trajectory of a pedal rotatably attached to the bicycle frame, and a distance between the saddle and the pedal, and load data representing the detected load applied to the pedal by the cyclist, the load data including pedaling force data detected by the detection section (14);
> employing in a joint torque estimation section (124) the skeletal data, the structural data, and data in the load data corresponding to at least one revolution of the pedal to estimate (S110) a trajectory of a joint of the cyclist for the one revolution of the pedal when the cyclist is seated on the saddle in an initial position, and using inverse dynamic analysis to estimate (S112) joint torque for the respective joints of the cyclist based on an estimated motion of the cyclist;
> in a change estimation section (126):

>> setting the position of the saddle for when displaced by a predetermined amount from the current position;
>> estimating the motion of the cyclist for a single revolution of the pedal with the position of the saddle displaced by the predetermined amount;
>> employing the data acquired for the pedaling force during a single revolution of the pedal to compute changes in joint torque,
>> computing a predetermined performance measure to evaluate pedaling performance,
>> executing a convergence test on the performance measure to determine whether or not the performance measure is the maximum value out of performance measures derived thus far, and, when affirmative determination is made that the test result is at a maximum, transitioning to the following step, but when determination is negative, returning to the previous step of setting the position of the saddle, in which case the processing is executed with the position of the saddle displaced by a predetermined amount from its current position, and
>> deciding the position of the saddle corresponding to the joint torque change giving the maximum value of the performance measure as the height of the saddle enabling the cyclist to develop their maximum power; and

> displaying in an output section (18) the decided height of the saddle enabling the cyclist to develop their maximum power.

**7.** A non-volatile storage medium storing computer program instructions for execution by a processor, the computer program instructions, when executed by the processor causing the processor to:

> detect in a detection section (14) load and pedaling force applied to a pedal by a cyclist;
> acquire skeletal data representing a skeletal structure of a cyclist including a position of joints of the cyclist and an inter-joint distance, structural data representing a structure of a bicycle and including an initial position of a saddle displaceably attached to a bicycle frame, a trajectory of a pedal rotatably attached to the bicycle frame, and a distance between the saddle and the pedal, and load data representing the detected load applied to the pedal by the cyclist, the load data including pedaling force data detected by the detection section (14);
> employ the skeletal data, the structural data, and data in the load data corresponding to at least one revolution of the pedal to estimate a trajectory of a joint of the cyclist for the one revolution of the pedal when the cyclist is seated on the saddle in an initial position, and use inverse dynamic analysis to estimate joint torque for the respective joints of the cyclist based on an estimated motion of the cyclist;
> set the position of the saddle for when displaced by a predetermined amount from the current position;
> estimate the motion of the cyclist for a single revolution of the pedal with the position of the saddle displaced by the predetermined amount;

employ the data acquired for the pedaling force during a single revolution of the pedal to compute changes in joint torque,

compute a predetermined performance measure to evaluate pedaling performance,

execute a convergence test on the performance measure to determine whether or not the performance measure is the maximum value out of performance measures derived thus far, and, when affirmative determination is made that the test result is at a maximum, transition to the following step, but when determination is negative, return to the previous step of setting the position of the saddle, in which case the processing is executed with the position of the saddle displaced by a predetermined amount from its current position, and

decide the position of the saddle corresponding to the joint torque change giving the maximum value of the performance measure as the height of the saddle enabling the cyclist to develop their maximum power; and

display in an output section (18) the decided height of the saddle enabling the cyclist to develop their maximum power.

**Patentansprüche**

1.  Gelenkdrehmoment-Berechnungssystem (10), Folgendes umfassend:

    einen Ermittlungsabschnitt (14), der dafür konfiguriert ist, die durch eine radfahrende Person auf ein Pedal ausgeübte Last und Pedalierkraft zu ermitteln;

    einen Erfassungsabschnitt (122), der dafür konfiguriert ist, Folgendes zu erfassen:

    Skelettdaten, die eine Skelettstruktur einer radfahrenden Person darstellen, einschließlich einer Position von Gelenken der radfahrenden Person und eines Abstandes zwischen Gelenken,

    Strukturdaten, die eine Struktur eines Fahrrades darstellen und eine Anfangsposition eines Sattels, der verschiebbar an einem Fahrradrahmen angebracht ist, eine Trajektorie eines Pedals, das drehbar an dem Fahrradrahmen angebracht ist, und einen Abstand zwischen dem Sattel und dem Pedal einschließen, und

    Lastdaten, welche die durch den Ermittlungsabschnitt (14) ermittelte Last darstellen, wobei die Lastdaten durch den Ermittlungsabschnitt (14) ermittelte Pedalierkraftdaten einschließen;

    einen Gelenkdrehmoment-Schätzungsabschnitt (124), der dafür konfiguriert ist, die Skelettdaten, die Strukturdaten und Daten in den Lastdaten, die mindestens einer Umdrehung des Pedals entsprechen, einzusetzen, um eine Trajektorie eines Gelenks der radfahrenden Person für die eine Umdrehung des Pedals zu schätzen, wenn die radfahrende Person in einer Anfangsposition auf dem Sattel sitzt, und eine inverse dynamische Analyse zu verwenden, um ein Gelenkdrehmoment für die jeweiligen Gelenke der radfahrenden Person auf der Grundlage einer geschätzten Bewegung der radfahrenden Person zu schätzen;

    einen Änderungsschätzungsabschnitt (126), der dafür konfiguriert ist:

    die Position des Sattels einzustellen, wenn er um einen vorbestimmten Betrag von der aktuellen Position verschoben ist;

    die Bewegung der radfahrenden Person für eine einzelne Umdrehung des Pedals zu schätzen, wenn die Position des Sattels um den vorbestimmten Betrag verschoben ist;

    die für die Pedalierkraft während einer einzelnen Umdrehung des Pedals erfassten Daten einzusetzen, um Änderungen eines Gelenkdrehmoments zu berechnen,

    ein vorbestimmtes Leistungsfähigkeitsmaß zu berechnen, um die Pedalierleistungsfähigkeit zu bewerten,

    einen Konvergenztest auf dem Leistungsfähigkeitsmaß auszuführen, um zu bestimmen, ob das Leistungsfähigkeitsmaß der maximale Wert von bisher abgeleiteten Leistungsmaßen ist oder nicht, und wenn die Bestimmung positiv ausfällt, dass das Testergebnis an einem Maximum ist, zum folgenden Schritt überzugehen, aber wenn die Bestimmung negativ ist, zum vorhergehenden Schritt des Einstellens der Position des Sattels zurückzukehren, in welchem Fall die Verarbeitung mit der Position des Sattels ausgeführt wird, die um einen vorbestimmten Betrag von seiner aktuellen Position verschoben ist, und

    die Position des Sattels, die der Gelenkdrehmomentänderung entspricht, die den Maximalwert des Leistungsfähigkeitsmaßes ergibt, als die Höhe des Sattels festzulegen, die es der radfahrenden Person ermöglicht, ihre maximale Leistung zu entwickeln; und

    einen Ausgabeabschnitt (18), der dafür konfiguriert ist, die durch den Änderungsschätzungsabschnitt (126) abgeleitete festgelegte Höhe des Sattels anzuzeigen, die es der radfahrenden Person ermöglicht, ihre maximale Leistung zu entwickeln.

2. Gelenkdrehmoment-Berechnungssystem (10) nach Anspruch 1, wobei das Leistungsfähigkeitsmaß eine Funktion ist, die einen Belastungsquotienten von Gelenkleistung darstellt, der auf der Grundlage des Gelenkdrehmoments und einer Gelenkwinkelgeschwindigkeit in Bezug auf die auf das Pedal ausgeübte Last abgeleitet ist.

3. Gelenkdrehmoment-Berechnungssystem (10) nach Anspruch 1 oder Anspruch 2, wobei:

Positionen der Gelenke der radfahrenden Person Positionen eines Hüftgelenks, eines Kniegelenks und eines Fußgelenks der radfahrenden Person einschließen; und
der Gelenkdrehmoment-Schätzungsabschnitt (124) dafür konfiguriert ist, ein Gelenkdrehmoment für mindestens ein Gelenk von Folgendem zu schätzen: das Hüftgelenk, das Kniegelenk oder das Fußgelenk.

4. Gelenkdrehmoment-Berechnungssystem (10) nach Anspruch 3, wobei ein Gelenkdrehmoment unter Verwendung eines Radfahrermodells geschätzt wird, in dem die radfahrende Person in einem Zustand, in dem sie das Fahrrad fährt, modelliert wird, wobei Stellen, die das Hüftgelenk, das Kniegelenk und das Fußgelenk darstellen, als Knoten modelliert sind, und Stellen der radfahrenden Person, welche die jeweiligen Knoten des Hüftgelenks, des Kniegelenks und des Fußgelenks verbinden, als Verbindungen modelliert sind.

5. Gelenkdrehmoment-Berechnungssystem (10) nach einem der Ansprüche 1 bis 4, wobei der Erfassungsabschnitt (122) dafür konfiguriert ist, die Skelettdaten und die Strukturdaten zu erfassen, die in einem Speicherabschnitt gespeichert worden sind.

6. Gelenkdrehmoment-Berechnungsverfahren, Folgendes umfassend:

in einem Ermittlungsabschnitt (14), Ermitteln der durch eine radfahrende Person auf ein Pedal ausgeübten Last und Pedalierkraft;
in einem Erfassungsabschnitt (122), Erfassen (S100, S102) von Skelettdaten, die eine Skelettstruktur einer radfahrenden Person darstellen, einschließlich einer Position von Gelenken der radfahrenden Person und eines Abstandes zwischen Gelenken, von Strukturdaten, die eine Struktur eines Fahrrades darstellen und eine Anfangsposition eines Sattels, der verschiebbar an einem Fahrradrahmen angebracht ist, eine Trajektorie eines Pedals, das drehbar an dem Fahrradrahmen angebracht ist, und einen Abstand zwischen dem Sattel und dem Pedal einschließen, und von Lastdaten, welche die ermittelte Last darstellen, die durch die radfahrende Person auf das Pedal ausgeübt wird, wobei die Lastdaten durch den Ermittlungsabschnitt (14) ermittelte Pedalierkraftdaten einschließen;
in einem Gelenkdrehmoment-Schätzungsabschnitt (124), Einsetzen der Skelettdaten, der Strukturdaten und der Daten in den Lastdaten, die mindestens einer Umdrehung des Pedals entsprechen, um eine Trajektorie eines Gelenks der radfahrenden Person für die eine Umdrehung des Pedals abzuschätzen (S110), wenn die radfahrende Person in einer Anfangsposition auf dem Sattel sitzt, und Verwenden einer inversen dynamischen Analyse, um ein Gelenkdrehmoment für die jeweiligen Gelenke der radfahrenden Person auf der Grundlage einer geschätzten Bewegung der radfahrenden Person abzuschätzen (S112);
in einem Änderungsschätzungsabschnitt (126):

Einstellen der Position des Sattels, wenn er um einen vorbestimmten Betrag von der aktuellen Position verschoben ist;
Schätzen der Bewegung der radfahrenden Person für eine einzelne Umdrehung des Pedals, wobei die Position des Sattels um den vorbestimmten Betrag verschoben ist;
Einsetzen der für die Pedalierkraft während einer einzelnen Umdrehung des Pedals erfassten Daten, um Änderungen eines Gelenkdrehmoments zu berechnen,
Berechnen eines vorbestimmten Leistungsfähigkeitsmaßes, um eine Pedalierleistungsfähigkeit zu bewerten,
Ausführen eines Konvergenztests auf dem Leistungsfähigkeitsmaß, um zu bestimmen, ob das Leistungsfähigkeitsmaß der Maximalwert von bisher abgeleiteten Leistungsfähigkeitsmaßen ist oder nicht, und wenn die Bestimmung positiv ist, dass das Testergebnis ein Maximum ist, Übergehen zum folgenden Schritt, aber wenn die Bestimmung negativ ist, Zurückkehren zum vorherigen Schritt des Einstellens der Position des Sattels, in welchem Fall die Verarbeitung mit der Position des Sattels ausgeführt wird, die um einen vorbestimmten Betrag von seiner aktuellen Position verschoben ist, und
Festlegen derjenigen Position des Sattels, die der Gelenkdrehmomentänderung entspricht, die den maximalen Wert des Leistungsfähigkeitsmaßes ergibt, als die Höhe des Sattels, die es der radfahrenden Person ermöglicht, ihre maximale Leistung zu entwickeln; und

in einem Ausgabeabschnitt (18), Anzeigen der festgelegten Höhe des Sattels, die es der radfahrenden Person ermöglicht, ihre maximale Leistung zu entwickeln.

**7.** Nichtflüchtiges Speichermedium, das Computerprogrammanweisungen zur Ausführung durch einen Prozessor speichert, wobei die Computerprogrammanweisungen, wenn sie durch den Prozessor ausgeführt werden, den Prozessor dazu veranlassen:

in einem Ermittlungsabschnitt (14) die durch eine radfahrende Person auf ein Pedal ausgeübte Last und Pedalierkraft zu ermitteln;

Skelettdaten, die eine Skelettstruktur einer radfahrenden Person darstellen, einschließlich einer Position von Gelenken der radfahrenden Person und eines Abstandes zwischen Gelenken, Strukturdaten, die eine Struktur eines Fahrrades darstellen und eine Anfangsposition eines Sattels, der verschiebbar an einem Fahrradrahmen angebracht ist, eine Trajektorie eines Pedals, das drehbar an dem Fahrradrahmen angebracht ist, und einen Abstand zwischen dem Sattel und dem Pedal einschließen, und Lastdaten, welche die ermittelte Last darstellen, die durch die radfahrende Person auf das Pedal ausgeübt wird, wobei die Lastdaten durch den Ermittlungsabschnitt (14) ermittelte Pedalierkraftdaten einschließen, zu erfassen;

die Skelettdaten, die Strukturdaten und Daten in den Lastdaten, die mindestens einer Umdrehung des Pedals entsprechen, einzusetzen, um eine Trajektorie eines Gelenks der radfahrenden Person für die eine Umdrehung des Pedals zu schätzen, wenn die radfahrende Person in einer Anfangsposition auf dem Sattel sitzt, und eine inverse dynamische Analyse zu verwenden, um ein Gelenkdrehmoment für die jeweiligen Gelenke der radfahrenden Person auf der Grundlage einer geschätzten Bewegung der radfahrenden Person zu schätzen;

die Position des Sattels einzustellen, wenn er um einen vorbestimmten Betrag von der aktuellen Position verschoben ist;

die Bewegung der radfahrenden Person für eine einzelne Umdrehung des Pedals zu schätzen, wobei die Position des Sattels um den vorbestimmten Betrag verschoben ist;

die für die Pedalierkraft während einer einzelnen Umdrehung des Pedals erfassten Daten einzusetzen, um Änderungen eines Gelenkdrehmoments zu berechnen,

ein vorbestimmtes Leistungsfähigkeitsmaß zu berechnen, um eine Pedalierleistungsfähigkeit zu bewerten,

einen Konvergenztest auf dem Leistungsfähigkeitsmaß auszuführen, um zu bestimmen, ob das Leistungsfähigkeitsmaß der maximale Wert von bisher abgeleiteten Leistungsfähigkeitsmaßen ist oder nicht, und wenn die Bestimmung positiv ausfällt, dass das Testergebnis an einem Maximum ist, zum folgenden Schritt überzugehen, aber wenn die Bestimmung negativ ist, zum vorhergehenden Schritt des Einstellens der Position des Sattels zurückzukehren, in welchem Fall die Verarbeitung mit der Position des Sattels ausgeführt wird, die um einen vorbestimmten Betrag von seiner aktuellen Position verschoben ist, und

die Position des Sattels, die der Gelenkdrehmomentänderung entspricht, die den Maximalwert des Leistungsfähigkeitsmaßes ergibt, als die Höhe des Sattels festzulegen, die es der radfahrenden Person ermöglicht, ihre maximale Leistung zu entwickeln; und

in einem Ausgabeabschnitt (18) die festgelegte Höhe des Sattels anzuzeigen, die es der radfahrenden Person ermöglicht, ihre maximale Leistung zu entwickeln.

## Revendications

**1.** Système de calcul de couple d'articulation (10), comprenant :
une section de détection (14) configurée pour détecter une charge et une force de pédalage appliquées à une pédale par un cycliste :

une section d'acquisition (122) configurée pour acquérir
des données squelettiques représentant une structure squelettique d'un cycliste, y compris une position d'articulations du cycliste et une distance inter-articulaire,
des données structurelles représentant une structure d'une bicyclette et incluant une position initiale d'une selle fixée de manière déplaçable à un cadre de bicyclette, une trajectoire d'une pédale fixée de manière rotative au cadre de bicyclette, et une distance entre la selle et la pédale, et
des données de charge représentant la charge détectée par la section de détection (14), les données de charge incluant des données de force de pédalage détectées par la section de détection (14),
une section d'estimation de couple d'articulation (124) configurée pour employer les données squelettiques, les données structurelles et des données dans les données de charge correspondant à au moins un tour de la pédale, pour estimer une trajectoire d'une articulation du cycliste pour le tour de la pédale lorsque le cycliste est assis sur la selle dans une position initiale, et pour utiliser une analyse dynamique inverse pour estimer un couple d'articulation pour les articulations respectives du cycliste sur la base d'un mouvement estimé du cycliste ; une section d'estimation de changement (126) configurée pour :

régler la position de la selle pour un cas où elle est déplacée d'une quantité prédéterminée par rapport à la position en cours ;

estimer le mouvement du cycliste pour un unique tour de la pédale avec la position de la selle déplacée de la quantité prédéterminée ;

employer les données acquises pour la force de pédalage au cours d'un unique tour de la pédale pour calculer des changements dans un couple d'articulation,

calculer une mesure de performance prédéterminée pour évaluer une performance de pédalage,

exécuter un test de convergence sur la mesure de performance pour déterminer si la mesure de performance correspond ou non à la valeur maximale parmi des mesures de performance dérivées jusqu'à présent et, en cas de détermination positive selon laquelle le résultat de test est à un maximum, passer à l'étape successive, mais en cas de détermination négative, revenir à l'étape précédente de réglage de la position de la selle, auquel cas le traitement est exécuté avec la position de la selle déplacée d'une quantité prédéterminée par rapport à sa position en cours, et

déterminer la position de la selle correspondant au changement de couple d'articulation donnant la valeur maximale de la mesure de performance comme étant la hauteur de la selle permettant au cycliste de développer sa puissance maximale ; et

une section de sortie (18) configurée pour afficher la hauteur déterminée de la selle permettant au cycliste de développer sa puissance maximale, dérivée de la section d'estimation de changement (126).

2. Système de calcul de couple d'articulation (10) selon la revendication 1, dans lequel la mesure de performance est une fonction représentant un quotient de contrainte de puissance articulaire dérivée sur la base du couple d'articulation et d'une vitesse angulaire articulaire par rapport à la charge appliquée à la pédale.

3. Système de calcul de couple d'articulation (10) selon la revendication 1 ou la revendication 2, dans lequel :

des positions des articulations du cycliste incluent des positions d'une articulation de hanche, d'une articulation de genou et d'une articulation de cheville du cycliste ; et

la section d'estimation de couple d'articulation (124) est configurée pour estimer un couple d'articulation pour au moins une articulation parmi l'articulation de hanche, l'articulation de genou ou l'articulation de cheville.

4. Système de calcul de couple d'articulation (10) selon la revendication 3, dans lequel le couple d'articulation est estimé en utilisant un modèle de cycliste dans lequel le cycliste, dans un état en train de rouler à bicyclette, est modélisé avec des sites représentant l'articulation de hanche, l'articulation de genou et l'articulation de cheville modélisées en tant que des noeuds, et des sites du cycliste reliant les noeuds respectifs de l'articulation de hanche, de l'articulation de genou et de l'articulation de cheville modélisés en tant que des liens.

5. Système de calcul de couple d'articulation (10) selon l'une quelconque des revendications 1 à 4, dans lequel la section d'acquisition (122) est configurée pour acquérir les données squelettiques et les données structurelles qui ont été stockées dans une section de stockage.

6. Procédé de calcul de couple d'articulation, comprenant les étapes consistant à :

détecter, dans une section de détection (14), une charge et une force de pédalage appliquées à une pédale par un cycliste ;

acquérir (S100, S102), dans une section d'acquisition (122), des données squelettiques représentant une structure squelettique d'un cycliste, incluant une position d'articulations du cycliste et une distance inter-articulaire, des données structurelles représentant une structure d'une bicyclette et incluant une position initiale d'une selle fixée de manière déplaçable à un cadre de bicyclette, une trajectoire d'une pédale fixée de manière rotative au cadre de bicyclette, et une distance entre la selle et la pédale, et des données de charge représentant la charge détectée appliquée à la pédale par le cycliste, les données de charge incluant des données de force de pédalage détectées par la section de détection (14) ;

employer, dans une section d'estimation de couple d'articulation (124), les données squelettiques, les données structurelles et des données dans les données de charge correspondant à au moins un tour de la pédale, pour estimer (S110) une trajectoire d'une articulation du cycliste pour le tour de la pédale lorsque le cycliste est assis sur la selle dans une position initiale, et utiliser une analyse dynamique inverse pour estimer (S112) un couple d'articulation pour les articulations respectives du cycliste sur la base d'un mouvement estimé du cycliste ;

dans une section d'estimation de changement (126) :

régler la position de la selle pour un cas où elle est déplacée d'une quantité prédéterminée par rapport à la position en cours ;

estimer le mouvement du cycliste pour un unique tour de la pédale avec la position de la selle déplacée de la quantité prédéterminée ;

employer les données acquises pour la force de pédalage au cours d'un unique tour de la pédale pour calculer des changements dans un couple d'articulation,

calculer une mesure de performance prédéterminée pour évaluer une performance de pédalage,

exécuter un test de convergence sur la mesure de performance pour déterminer si la mesure de performance correspond ou non à la valeur maximale parmi des mesures de performance dérivées jusqu'à présent, et, en cas de détermination positive selon laquelle le résultat de test est à un maximum, passer à l'étape successive, mais en cas de détermination négative, revenir à l'étape précédente de réglage de la position de la selle, auquel cas le traitement est exécuté avec la position de la selle déplacée d'une quantité prédéterminée par rapport à sa position en cours, et

déterminer la position de la selle correspondant au changement de couple d'articulation donnant la valeur maximale de la mesure de performance comme étant la hauteur de la selle permettant au cycliste de développer sa puissance maximale ; et

afficher, dans une section de sortie (18), la hauteur déterminée de la selle permettant au cycliste de développer sa puissance maximale.

7. Support de stockage non volatile stockant des instructions de programme informatique destinées à être exécutées par un processeur, les instructions de programme informatique, lorsqu'elles sont exécutées par le processeur, amenant le processeur à :

détecter, dans une section de détection (14), une charge et une force de pédalage appliquées à une pédale par un cycliste ;

acquérir des données squelettiques représentant une structure squelettique d'un cycliste, incluant une position d'articulations du cycliste et une distance inter-articulaire, des données structurelles représentant une structure d'une bicyclette et incluant une position initiale d'une selle fixée de manière déplaçable à un cadre de bicyclette, une trajectoire d'une pédale fixée de manière rotative au cadre de bicyclette, et une distance entre la selle et la pédale, et des données de charge représentant la charge détectée appliquée à la pédale par le cycliste, les données de charge incluant des données de force de pédalage détectées par la section de détection (14) ;

employer les données squelettiques, les données structurelles et des données dans les données de charge correspondant à au moins un tour de la pédale, pour estimer une trajectoire d'une articulation du cycliste pour le tour de la pédale lorsque le cycliste est assis sur la selle dans une position initiale, et utiliser une analyse dynamique inverse pour estimer un couple d'articulation pour les articulations respectives du cycliste sur la base d'un mouvement estimé du cycliste ;

régler la position de la selle pour un cas où elle est déplacée d'une quantité prédéterminée par rapport à la position en cours ;

estimer le mouvement du cycliste pour un unique tour de la pédale avec la position de la selle déplacée de la quantité prédéterminée ;

employer les données acquises pour la force de pédalage au cours d'un unique tour de la pédale pour calculer des changements dans un couple d'articulation,

calculer une mesure de performance prédéterminée pour évaluer une performance de pédalage,

exécuter un test de convergence sur la mesure de performance pour déterminer si la mesure de performance correspond ou non à la valeur maximale parmi des mesures de performance dérivées jusqu'à présent et, en cas de détermination positive selon laquelle le résultat de test est à un maximum, passer à l'étape successive, mais en cas de détermination négative, revenir à l'étape précédente de réglage de la position de la selle, auquel cas le traitement est exécuté avec la position de la selle déplacée d'une quantité prédéterminée par rapport à sa position en cours, et

déterminer la position de la selle correspondant au changement de couple d'articulation donnant la valeur maximale de la mesure de performance comme étant la hauteur de la selle permettant au cycliste de développer sa puissance maximale ; et

afficher, dans une section de sortie (18), la hauteur déterminée de la selle permettant au cycliste de développer sa puissance maximale.

FIG.1

10

12

40    14    122    124    126    18

CYCLIST    DETECTION SECTION    DATA ACQUISITION SECTION    TORQUE ESTIMATION SECTION    CHANGE ESTIMATION SECTION    OUTPUT SECTION

INPUT SECTION (STORAGE SECTION)    16

EP 3 560 808 B1

FIG.2

# FIG.3

FIG.4

# FIG.5

FIG.6

## FIG.7A

HIP JOINT
TORQUE

O

O          180°          ROTATION ANGLE

## FIG.7B

HIP JOINT
ANGULAR
VELOCITY

O

O          180°          ROTATION ANGLE

## FIG.7C

HIP JOINT
POWER

O

O          180°          ROTATION ANGLE

## FIG.8

FIG.9

19(10)

13(12)

13A — CPU

13B — RAM

13C — ROM

13P — COMPUTATION PROGRAM

CONTROL SECTION

I / O

13E  13D

DETECTION SECTION — 14

NON-VOLATILE MEMORY — 15

INPUT SECTION — 16

OUTPUT SECTION — 18

# FIG.10

START

ACQUIRE STRUCTURAL DATA
(MODEL ANALYSIS SUBJECT) — S100

ACQUIRE PEDALING FORCE DISTRIBUTION
FOR SINGLE REVOLUTION OF PEDAL — S102

ESTIMATE MOTION FOR SINGLE
REVOLUTION OF PEDAL — S110

ESTIMATE TORQUE DISTRIBUTION OF
EACH JOINT USING INVERSE DYNAMICS — S112

OPTIMIZATION PROCESSING
(CHANGE COMPUTATION) — S120

SET SADDLE POSITION DISPLACED BY
PREDETERMINED AMOUNT FROM CURRENT POSITION — S121

ESTIMATE MOTION FOR SINGLE
REVOLUTION OF PEDAL — S122

COMPUTE IN JOINT TORQUE AMOUNT
USING ACQUIRED PEDALING FORCE DISTRIBUTION — S123

COMPUTE PERFORMANCE MEASURE — S124

CONVERGENCE TEST — S125

N  CONVERGED (MAXIMUM)? — S126

Y

DECIDE SADDLE POSITION — S127

OUTPUT DATA — S128

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005527004 A
- JP 2015011714 A
- JP 2014008789 A

- JP 2016107093 A
- JP 2015091311 A

**Non-patent literature cited in the description**

- **YUNG-SHENG LIU et al.** Muscles force and joints load simulation of bicycle riding using multibody models. *PROCEDIA ENGINEERING,* vol. 13, ISSN ISSN: 1877-7058, 81-87

- **MICHIYOSHI AE ; HAIPENG TANG ; TAKASHI YOKOI.** Estimation of Inertia Properties of Body Segments in Japanese Athletes (forms and kinematic measurement). *Biomechanisms,* 1992, vol. 11, 23-33